Europäisches Patentamt

European Patent Office

Office européen des brevets

(19)

(11) Veröffentlichungsnummer: **0 026 477**
**B1**

(12) **EUROPÄISCHE PATENTSCHRIFT**

(45) Veröffentlichungstag der Patentschrift:
02.01.85

(21) Anmeldenummer: 80105827.2

(22) Anmeldetag: 25.09.80

(51) Int. Cl.³: **A 61 N 1/36**, H 03 K 3/00

(54) **Herzschrittmacher.**

(30) Priorität: 27.09.79 DE 2939174

(43) Veröffentlichungstag der Anmeldung:
08.04.81 Patentblatt 81/14

(45) Bekanntmachung des Hinweises auf die Patenterteilung:
02.01.85 Patentblatt 85/1

(84) Benannte Vertragsstaaten:
**DE FR NL SE**

(56) Entgegenhaltungen:
**AT - B - 352 261**
**DE - A - 2 327 602**

(73) Patentinhaber: **Siemens Aktiengesellschaft, Berlin und München Wittelsbacherplatz 2, D-8000 München 2 (DE)**

(72) Erfinder: **Elmqvist, Häkan, Dr., Sunnerdahisvägen 7, S-161 38 Bromma (SE)**
Erfinder: **Ekwall, Christer, Jungfrudansen 9, S-171 56 Solna (SE)**

## Beschreibung

Die Erfindung bezieht sich auf einen Herzschrittmacher mit batteriegespeistem Oszillator, insbesondere Quarzoszillator, zur Vorgabe einer internen Taktfrequenz, sowie gegebenenfalls mit Frequenzuntersetzer zur Untersetzung der internen Taktfrequenz in Richtung Herzschrittmacherfrequenz.

Herzschrittmacher sollen weitgehend frequenzstabil sein, und sie sollen dabei gleichzeitig möglichst geringe Verlustleistung aufweisen. Letzteres gilt insbesondere für implantierbare batteriegespeiste Herzschrittmacher, die zur Erzielung hoher Lebensdauer der Batterie verlustarm ausgelegt sein sollen. Eine recht gute Frequenzstabilität wird üblicherweise erreicht durch Einsetzung von Quarzoszillatoren. Die Schwingquarze solcher Quarzoszillatoren sind jedoch bei niedrigen Frequenzen relativ voluminös. Aus diesem Grunde werden bevorzugt höherfrequente und damit kleinvolumigere Schwingquarze eingesetzt. Höherfrequente Schwingquarze haben jedoch höheren Energiebedarf. Die Batterie wird in unnötiger Weise zu stark belastet.

Aus der DE-A-2 327 602 ist ein Herzschrittmacher bekannt, bei dem der zeitliche Klemmenspannungsabfall einer Nuklearbatterie durch ein dem Eingang eines Rechteckimpulsgenerators vorgeschaltetes Konstantstromelement ausgeglichen wird, wodurch zusätzlich auch eine Verringerung des Energieumsatzes im Schrittmacher bewirkt wird.

Aufgabe vorliegender Erfindung ist es, einen Herzschrittmacher der eingangs genannten Art dahingehend auszubilden, daß einerseits gute Frequenzstabilität gewährleistet ist und andererseits aber auch gleichzeitig die Verlustleistung optimal gering ist.

Diese Aufgabe wird erfindungsgemäß dadurch gelöst, daß im batteriegespeisten, den Hochfrequenzstrom führenden Teil des Quarzoszillators ein Strombegrenzer zur Begrenzung der Verlustleistung eingeschaltet ist.

Die Erfindung geht von der Erkenntnis aus, daß der Spannungsbedarf des Oszillators mit Beginn des Schwingens sich zunehmend zu höheren Werten reguliert. Hierdurch wird der Batterie zunehmend Strom entnommen, was schließlich zu den unerwünschten Energieverlusten führt. Die Erfindung verwendet einen Strombegrenzer im batteriegespeisten stromführenden Teil des Oszillators; hierdurch wird erreicht, daß dem Anstieg des Stromes bei ansteigender Spannung Grenzen gesetzt werden. Dementsprechend wird dann auch die Verlustleistung auf einen vorgewählten Wert begrenzt.

In vorteilhafter Ausgestaltung der Erfindung soll der Strombegrenzer ein Konstantstromelement sein (z. B. in Ausbildung als integrierter Transistor). Weitere vorteilhafte Ausgestaltungen ergeben sich aus den restlichen Unteransprüchen.

Im folgenden wird ein Ausführungsbeispiel der Erfindung an Hand zweier Figuren näher beschrieben. Es zeigt

Fig. 1 die Erfindung im Prinzipschaltbild,

Fig. 2 eine vorteilhafte integrierbare Ausführungsform des Prinzipschaltbildes der Fig. 1.

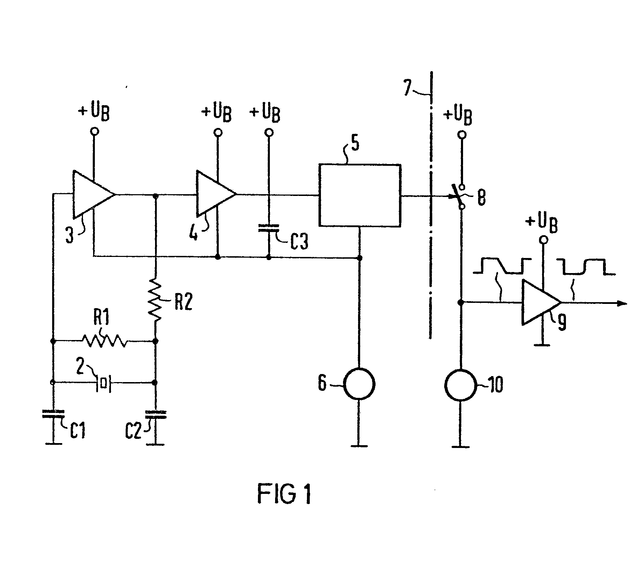

In der Fig. 1 ist ein Quarzoszillator eines Herzschrittmachers dargestellt, der einen Schwingquarz 2 (z. B. Frequenz $f_0$ ca. 32 kHz), Beschaltungskapazitäten C1 und C2 (z. B. ca. 15 pF) und Widerstände R1 und R2 (z. B. R1 ca. 22 M$\Omega$ und R2 ca. 330 k$\Omega$) umfaßt. Ein im Batteriekreis ($U_B$) liegender Verstärker hat die Kennziffer 3. Auf den Verstärker 3 folgt ein Pulsformer 4 und darauf ein Frequenzteiler 5, der die Oszillatorfrequenz auf ca. 4 kHz heruntersetzt. Die Kapazität C3 weist einen Wert im Bereich 10 pF auf. Wesentliches erfinderisches Merkmal ist, daß in den batteriegespeisten stromführenden Teilen der Anordnung aus Oszillator, Pulsformer und Frequenzteiler als Strombegrenzer ein Konstantstromelement 6 eingeschaltet ist. Das Konstantstromelement 6 erzeugt einen Konstantstrom von ca. 0,5 µA. Es bewirkt, daß auch im Extremfall der Belastung in den batteriegespeisten stromführenden Teilen der Bauteile 3, 4, 5 höchstens ein Strom von 0,5 µA fließt. Der Gesamtverlust bei Batteriebelastung wird damit auf ein Minimum begrenzt.

In der Fig. 1 folgt auf die 4-kHz-Schnittstelle 7 ein Expander, der die 4-kHz-Pulskurve zur Weiterverarbeitung in Richtung Herzschrittmacherfrequenz in der Amplitude zu voller Spannung (in Richtung Batteriespannung) expandiert. Der Expander umfaßt den getakteten Schalter 8, das Verstärkungsteil 9 sowie ein weiteres Konstantstromelement 10, die dem höheren Bedarf entsprechend auf einen höheren Grenzstrom, von z. B. 1,5 µA, abgestellt ist.

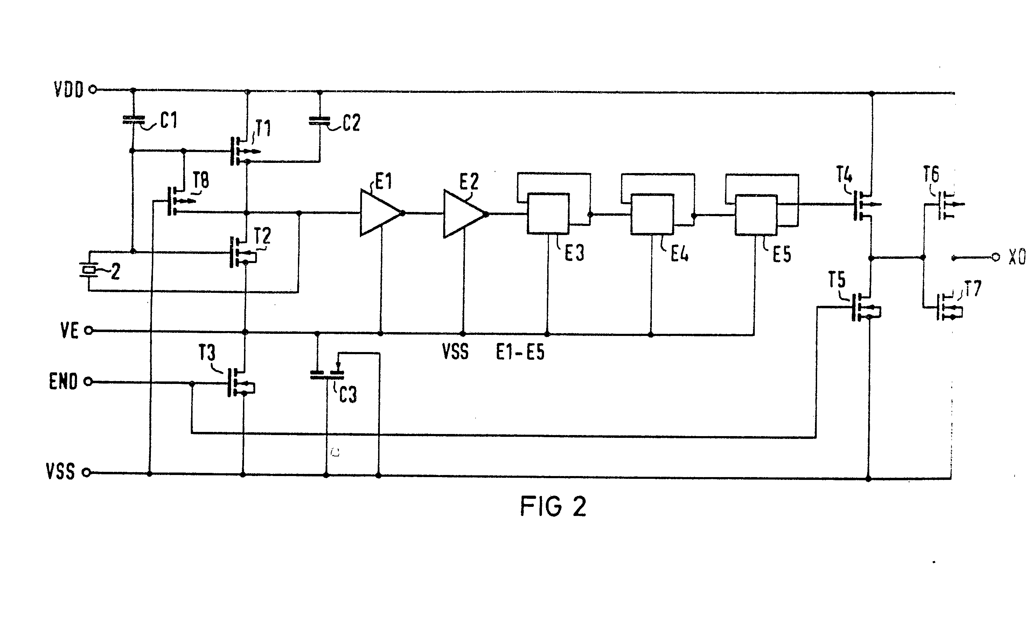

Die Fig. 2 zeigt eine integrierbare Ausführungsform des Prinzipschaltbildes der Fig. 1. Die aus der Batteriespannung $U_B$ jeweils abgeleiteten Versorgungsspannungen sind mit VDD, VE, END, VSS angegeben. Die Transistoren T1, T2 sind Bestandteil des Verstärkers 3 der Fig. 1. Der Transistor T3 bildet das Konstantstromelement 6. Der Transistor T8 entspricht in seiner Wirkung der des Widerstandes R1 des Oszillators der Fig. 1. E1, E2 bilden den Pulsformer 4, und E3 bis E5 formen den Frequenzteiler 5. T4 ist der Schalter 8, und T5 entspricht dem Konstantstromelement 10. T6 und T7 bilden schließlich den Verstärkungsteil 9 des Expanders.

## Patentansprüche

1. Herzschrittmacher mit batteriegespeistem Quarzoszillator zur Vorgabe einer internen Taktfrequenz sowie gegebenenfalls mit Frequenzuntersetzer zur Untersetzung der internen Taktfrequenz, dadurch gekennzeichnet, daß im batteriegespeisten, den Hochfrequenzstrom führenden Teil des Oszillators ein Strombegrenzer (6) zur Begrenzung der Verlustleistung eingeschaltet ist.

2. Herzschrittmacher nach Anspruch 1, dadurch gekennzeichnet, daß der Strombegrenzer (6) ein Konstantstromelement ist.

3. Herzschrittmacher nach Anspruch 1 oder 2, dadurch gekennzeichnet, daß dem Oszillator (1) gegebenenfalls nachgeschaltete weitere stromverzehrende Bauelemente, wie Pulsformer (4), Frequenzuntersetzer (5) oder dergleichen, ebenfalls im batteriegespeisten, den Hochfrequenzstrom führenden Teil einen Strombegrenzer aufweisen.

4. Herzschrittmacher nach einem der Ansprüche 1 bis 3, dadurch gekennzeichnet, daß sämtlichen Bauteilen (2—5) mit variierender Spannung ein erster Strombegrenzer (6) und nach einer Schnittstelle (7) zum Expandieren (8, 9) zur vollen Spannung dem Expander (9) ein weiterer Strombegrenzer (10) zugeordnet ist.

## Claims

1. A heart pacemaker having a battery-fed quartz oscillator for setting an internal clock pulse frequency and if necessary having frequency reducing means for reducing the internal clock pulse frequency, characterised in that a current limiter (6) for limiting the power loss is inserted into the battery-fed component of the oscillator which carries the high-frequency current.

2. A heart pacemaker as claimed in Claim 1, characterised in that the current limiter (6) is a constant current element.

3. A heart pacemaker as claimed in Claim 1 or 2, characterised in that further current-consuming components, which are if necessary connected following the oscillator (1), such as a pulse former (4), a frequency reducer (5) or the like, also have a current limiter in the battery-fed component which carries the high-frequency current.

4. A heart pacemaker as claimed in one of Claims 1 to 3, characterised in that all components (2—5) having a varying voltage are assigned a first current limiter (6) and after the point of intersection (7) for expansion means (8, 9) to achieve full voltage the expander (9) is assigned a further current limiter (10).

## Revendications

1. Stimulateur cardiaque à oscillateur piézoélectrique à alimentation par une pile en vue de donner à l'avance une fréquence de cadence interne et éventuellement à démultiplicateur de fréquence pour démultiplier la cadence de fréquence interne, caractérisé par le fait que dans la partie de l'oscillateur, alimentée par la pile et parcourue par le courant haute fréquence, est inséré un limiteur de courant (6) en vue de limiter la puissance dissipée.

2. Stimulateur cardiaque selon la revendication 1, caractérisé par le fait que le limiteur de courant (6) est un élément à courant constant.

3. Stimulateur cardiaque selon la revendication 1 ou 2, caractérisé par le fait que d'autres composants, tels qu'un conformateur d'impulsions (4), un démultiplicateur de fréquence (5) ou similaire, qui consomment du courant et qui sont éventuellement montés en aval de l'oscillateur (1), comportent également un limiteur de courant dont la partie alimentée par la pile est parcourue par le courant haute fréquence.

4. Stimulateur cardiaque selon l'une des revendications 1 à 3, caractérisé par le fait qu'à tous les éléments constitutifs (2—5) à tension variable est associé un premier limiteur de tension (6) et après un interface (7), pour l'expansion (8, 9) à la pleine tension, un limiteur de courant supplémentaire (10) est associé à l'expanseur (9).

FIG 1

FIG 2